# FASCICULE DE BREVET EUROPEEN

(11) **EP 1 996 633 B1**
(45) Date de publication et mention de la délivrance du brevet: **12.05.2010**
(21) Numéro de dépôt: 07731801.2
(22) Date de dépôt: 22.03.2007
(51) Int. Cl.: C08F 290/06, B01J 20/26, C07K 7/08, C08F 2/44, C12N 11/08

(54) **EMPREINTES MOLECULAIRES POUR UNE RECONNAISSANCE EN MILIEUX AQUEUX, LEURS PROCEDES DE PREPARATION ET LEURS UTILISATIONS**
MOLEKULARE PRÄGUNGEN ZUR ERKENNUNG IN WÄSSRIGEN MEDIEN, HERSTELLUNGSVERFAHREN DAFÜR UND VERWENDUNGEN DAVON
MOLECULAR IMPRINTINGS FOR RECOGNITION IN AQUEOUS MEDIA, METHODS FOR PREPARING SAME AND USES THEREOF

(30) Priorité: 22.03.2006 FR 0650992
(43) Date de publication de la demande: 03.12.2008
(73) Titulaire: Polyintell, 76000 Rouen (FR)
(72) Inventeur: NARAGHI, Kaynoush, F-76000 Rouen (FR); BAYOUDH, Sami, F-76000 Rouen (FR); AROTCARENA, Michel, F-76000 Rouen (FR)
(74) Mandataire: Leszczynski, André
(86) Numéro de dépôt international: PCT/FR2007/050988
(87) Numéro de publication internationale: WO 2007/107680

(56) Documents cités:
- ORAL EBRU ET AL: "Responsive and recognitive hydrogels using star polymers" J. BIOMED. MATER. RES. PART A; JOURNAL OF BIOMEDICAL MATERIALS RESEARCH - PART A FEB 1 2004, vol. 68, no. 3, 1 février 2004 (2004-02-01), pages 439-447, XP002394866
- TOMONORI NAKAYAMA: "Star Shaped Molecularly Imprinted Polymer Working as a Drug Carrier"[Online] juillet 2006 (2006-07), XP002452282 Extrait de l'Internet: URL:http://aiche.confex.com/aiche/2006/tec hprogram/P61653.HTM> [extrait le 2006-08-10]

## Description

La présente invention se rapporte au domaine des empreintes moléculaires utiles pour la reconnaissance de molécules cibles.

La présente invention vise en particulier des empreintes moléculaires sous la forme de nanosphères polymériques, réticulées, de type coeur-branches et plus particulièrement formées d'un cour hydrophobe et de branches de nature hydrophile.

La présente invention concerne également les nanogels et les hydrogels formés à partir de telles nanosphères polymériques, ainsi qu'à leurs utilisations.

Les matériaux à empreintes moléculaires, encore appelés MIPs en langue anglaise, sont obtenus par une technique d'impression moléculaire.

De façon générale, on obtient ces empreintes moléculaires en copolymérisant des monomères est agent(s) de réticulation en présence d'une molécule dont on cherche précisément à fixer l'empreinte. Les monomères s'arrangent spécifiquement autour de cette molécule, encore dénommée "molécule patron", par des interactions fortes ou faibles, puis sont polymérisés généralement en présence d'un taux élevé en agent de réticulation. Après polymérisation, la molécule est extraite du matériau polymère et laisse ainsi son empreinte moléculaire dans des cavités au sein du matériau. Ces cavités sont de réels récepteurs synthétiques comparables aux récepteurs biologiques de type anticorps. Des matériaux à empreintes moléculaires de type anticorps-artificiels ont été utilisés dans les applications de séparation en chromatographie, les capteurs, la catalyse de réaction chimique, l'extraction en phase solide, l'immunoanalyse, le criblage de librairies moléculaires...

Plus précisément, il existe deux approches possibles pour faire des empreintes moléculaires, l'approche covalente développée par Wulff dans le document US 4 127 730 et l'approche non covalente développée par Mosbach dans le document US 5 110 833.

Dans l'approche covalente, les interactions entre les monomères et la molécule patron sont de type liaisons covalentes labiles. Dans ce cas, après l'extraction de la molécule patron par rupture de la liaison covalente, la reconnaissance de molécules cibles s'effectue également par la formation d'une liaison covalente entre l'empreinte et la molécule cible considérée.

Dans l'approche non covalente de Mosbach, les interactions entre les monomères et la molécule patron sont des liaisons faibles de type liaisons hydrogène, liaisons ioniques, pi donneur - pi accepteur, liaisons de Van Der Waals ou interactions hydrophobiques. Après l'extraction de la molécule patron, la reconnaissance de molécules cibles s'effectue également par des interactions non covalentes, entre l'empreinte et la molécule cible.

Les deux approches peuvent être combinées en utilisant la première approche de type covalente pour la préparation du matériau à empreinte moléculaire et la deuxième approche pour obtenir une reconnaissance par des interactions non covalentes, comme cela est par exemple divulgué dans M.J. WHITCOMBE et al. "A New Method for the Introduction of Recognition Site Functionality into Polymers prepared by molecular Imprinting : Synthesis and Characterization of Polymeric Receptors for Cholesterol " J. Am. Chem. Soc., 1995, 117, 7105-7111.

Cette technique d'impression a montré son efficacité quand l'impression est réalisée dans des solvants organiques aprotiques mais, en revanche, a montré des faiblesse et des limitations quand l'impression est réalisée dans des solvants protiques polaires (eau, alcools).

Des tentatives ont été faites pour accroître la reconnaissance, notamment des molécules hydrophiles à l'image des sucres, dans l'eau ou dans des solvants protiques polaires, en mettant en oeuvre des interactions de type coordination métallique (Striegler et al. "Evaluation of new strategies to prépare templated polymers with sufficient oligosacharide récognition capacity", Analytica Chimica Acta 484, 2003, 53-62) sans toutefois donner entière satisfaction.

Egalement dans le cadre de la mise au point d'empreintes utiles pour une reconnaissance dans l'eau, des matrices très hydrophiles ont été préparées pour la riboflavine et ses dérivées dans le document WO 2004/067578, ou encore pour le D-glucose dans E.Oral et al. "Dynamic Studies of Molecular Imprinting Polymerizations", Polymer 45, 2004, 6163-6173.

Classiquement, les empreintes moléculaires sont synthétisées sous la forme d'un monolithe, par solubilisation de l'ensemble des composés dans un solvant jouant également le rôle de porogène. Les gels ainsi formés doivent ensuite être broyées et tamisées avant utilisation.

Pour s'affranchir de ces étapes de broyage et de tamisage, des méthodes reposant sur la polymérisation en milieu dispersé, et en particulier sous la forme d'une émulsion huile-dans-eau, ont par ailleurs été développées.

Parmi les techniques de polymérisation en milieu dispersé, certaines méthodes de précipitation utilisant des monomères solubles dans la phase continue ne nécessitent pas l'utilisation d'un agent tensio-actif (Lei Ye et al., Analytica Chimica Acta 435, 2001, 187-196). Ce type de méthode permet effectivement d'obtenir des microsphères uniformes avec un bon rendement, mais est en revanche limité par la dispersion de la molécule patron dans l'ensemble du mélange.

En fait, la plupart des méthodes par polymérisation en milieu dispersé nécessite généralement l'utilisation d'un agent tensioactif car les monomères ne sont pas solubles dans la phase continue. Ainsi, des exemples de polymérisation en suspension utilisant un agent tensioactif classique et une phase continue aqueuse (J. Matsui et al., Anal. Commun. 34, 1997, 85) ou une phase continue perfluorée (A.G. Mayes et al., Anal. Chem. 68, 1996, 3769) sont déjà bien connus. Dans le document US 2003/139483, il est par ailleurs proposé d'utiliser un agent tensioactif portant la molécule patron à l'une de ses extrémités.

On peut également citer le cas de la polymérisation en émulsion ensemencée (seed polymérisation) (K. Hosoya et al. in : R.A. Bartsch, M. Maeda (Eds.), "Molecular and Ionic récognition With Imprinted Polymers", American Chemical Society, Washingtion, DC, 1998, p. 143), la polymérisation par mini-émulsion (D Vaihinger et al., Macromol.Chem. Phys. 2002, 203, 1965-1973), la polymérisation "core-shell" (N. Perez et al., J. Appl. Polym. Sci 2000, 77, 1851-1859), ou encore la photopolymérisation UV (M.B. Mellott et al., Biomaterials 22, 2001, 929-941). Les agents tensioactifs utilisés selon ces méthodes s'absorbent sur la surface des particules polymériques par des interactions intermoléculaires physiques conduisant à la formation de micelles et à la réduction de la tension interfaciale des particules polymériques et donc à leur stabilisation.

Toutefois, la dispersion obtenue suivant ces procédés n'est pas toujours stable car l'équilibre d'absorption-désorption des agents tensioactifs entre la surface des particules et la phase continue peut être déplacé par suite d'une variation de certains paramètres tels le pH ou la température, entraînant selon les cas la précipitation des particules polymériques.

Il est connu, par Koide *et al.* d'utiliser un monomère fonctionnalisé à l'une de ces extrémités par une fonction carboxylique et à l'autre par une fonction vinylique pour la synthèse dans l'eau de résines de divinylbenzène complexant des ions métalliques (Y. Koide et al., Bull. Chem. Soc. Jpn. 1996, 69, 125). La fonction acide du monomère permet la complexation du métal à la surface de la résine et le caractère hydrophobe du monomère permet le maintien de cette fonction à la surface.

D'autres auteurs synthétisent des microgels solubles, qui sont des polymères réticulés en solution ayant des tailles suffisamment petites pour rester en solution malgré l'absence d'agents tensioactifs (Maddock et al. Chem . Commun . Novel imprinted soluble microgels with hydrolytic catalytic activity, 2004 , 536 - 537) (Biffis et al., Macromol. Chem. Phys. The Synthesis, Characterization and Molecular Recognition Properties of Imprinted Microgels 2001, 202, 163-171). Ces microgels sont synthétisés dans des solvants organiques tels que le diméthylsulfoxyde ou des cétones. Cette option ne s'avère donc appropriée que pour la préparation de particules polymériques de taille très réduite.

Il demeure donc un besoin d'une méthode permettant de stabiliser efficacement et durablement des particules polymériques obtenues par polymérisation en milieu dispersé, et qui soit dénuée des inconvénients précités.

La présente invention vise précisément à proposer une nouvelle méthode de polymérisation, utile pour la synthèse de matériaux à empreintes moléculaires appropriés à une reconnaissance en milieu aqueux, et conforme aux exigences précitées.

Ainsi, selon un premier de ses aspects, la présente invention concerne des nanosphères polymériques réticulées possédant une structure en étoile de type coeur-branches, dans laquelle les branches sont de nature hydrophile et le coeur est de nature polymérique, réticulé, hydrophobe et forme l'empreinte de tout ou partie au moins d'une molécule cible.

Selon un autre de ses aspects, l'invention concerne un procédé de préparation de nanosphères polymériques selon l'invention par copolymérisation, par exemple en milieu dispersé, d'au moins un macromonomère amphiphile avec au moins un monomère hydrophobe en présence d'au moins un agent de réticulation hydrophobe et d'au moins une molécule patron, suivie de l'extraction de ladite molécule patron hors desdites nanosphères ainsi formées, par exemple par lavage.

De manière inattendue, les inventeurs ont ainsi constaté que l'utilisation d'un macromonomère amphiphile, c'est-à-dire possédant au moins un motif, en particulier un segment, hydrophile et au moins un motif polymérisable hydrophobe, permet la synthèse de nanosphères polymériques amphiphiles comprenant des segments hydrophiles liés de façon covalente en surface d'un coeur hydrophobe.

Ainsi, selon un mode de réalisation particulier, l'invention concerne également des nanosphères polymériques réticulées possédant une structure en étoile de type coeur-branches dans laquelle les branches sont de nature hydrophile et respectivement formées par le segment hydrophile d'au moins au moins un macromonomère amphiphile, notamment tel que défini ci-dessus, et le coeur est de nature polymérique, réticulé, hydrophobe, forme l'empreinte de tout ou partie d'une molécule cible, et dérive de la copolymérisation, notamment radicalaire, du motif polymérisable hydrophobe dudit macromonomère amphiphile avec au moins un monomère hydrophobe en présence d'au moins un agent de réticulation hydrophobe et d'une molécule patron.

Selon un autre de ses aspects, l'invention concerne également un procédé de préparation de nanosphères polymériques selon l'invention comprenant :
- une première étape de copolymérisation d'au moins un monomère hydrophobe fonctionnalisé en présence d'au moins un agent de réticulation hydrophobe, d'au moins une molécule patron, et éventuellement d'au moins un monomère hydrophobe, notamment tel que défini ci-après,
- une deuxième étape de transformation en surface de l'entité polymérique hydrophobe et réticulée obtenue à l'issue de la première étape avec une ou plusieurs entités hydrophiles,
- et l'extraction de ladite molécule patron hors desdites nanosphères polymériques ainsi formées, par exemple par lavage.

Les nanosphères selon l'invention s'avèrent particulièrement avantageuses au regard de leur comportement amphiphile, lié à la présence concomitante au sein de leur structure d'un coeur hydrophobe, et de branches hydrophiles greffées de façon covalente audit coeur, solubles dans les milieux aqueux. Une telle organisation rend possible leur mise en dispersion et leur stabilisation en milieu aqueux par répulsion stérique entre les branches hydrophiles.

Une telle structure est également particulièrement avantageuse dans la mesure où elle garantit une stabilisation accrue de la dispersion des nanosphères ainsi obtenues, notamment sur une plus grande gamme de conditions opératoires, par exemple en terme de polarité du solvant ou de variations de pH.

Par ailleurs, lorsque des nanosphères polymériques selon l'invention sont conditionnées sous la forme d'une poudre, elles présentent également l'avantage de pouvoir être remises aisément en solution stable, sans ajout d'agents tensioactifs.

Un autre avantage des nanosphères polymériques selon l'invention est la stabilisation dans des milieux polaires et protiques tels que l'eau et les alcools des empreintes moléculaires formées au niveau de leur coeur hydrophobe. La reconnaissance de molécules cibles dans ces milieux y est par conséquent plus efficace.

Les nanosphères polymériques selon l'invention sont également utiles pour former des nanogels et des hydrogels.

Ainsi, selon un autre de ses aspects, l'invention concerne un nanogel se présentant sous la forme d'une dispersion de nanosphères polymériques conformes à l'invention

Un tel nanogel peut notamment être obtenu directement à l'issue des procédés de préparation des nanosphères polymériques selon l'invention proposés précédemment.

L'invention concerne donc également en particulier un procédé de préparation d'un nanogel de nanosphères polymériques selon l'invention comprenant au moins la copolymérisation, par exemple en milieu dispersé, d'au moins un macromonomère amphiphile comprenant un unique motif hydrophobe apte à copolymériser avec au moins un monomère hydrophobe en présence d'au moins un agent de réticulation hydrophobe et d'au moins une molécule patron, et l'extraction de ladite molécule patron hors desdites nanosphères polymériques ainsi formées, par exemple par lavage.

Selon encore un autre de ses aspects, l'invention concerne un hydrogel se présentant sous la forme d'un réseau de nanosphères polymériques conformes à l'invention.

L'invention concerne également un procédé de préparation d'un hydrogel de nanosphères polymériques selon l'invention comprenant au moins la copolymérisation, par exemple en milieu dispersé, d'au moins un macromonomère amphiphile comprenant au moins deux motifs hydrophobes aptes à copolymériser avec au moins un monomère hydrophobe en présence d'au moins un agent de réticulation hydrophobe et d'au moins une molécule patron, et l'extraction de ladite molécule patron hors desdites nanosphères polymériques ainsi formées, par exemple par lavage.

La présente invention a également pour objet l'utilisation des nanosphères polymériques, des nanogels et des hydrogels selon l'invention à des fins d'extraction, de détection, de séparation, de purification, d'absorption, d'adsorption, de rétention ou de libération contrôlée ou encore dans des applications choisies parmi les capteurs, la catalyse de réactions chimiques, le criblage de molécules, la synthèse chimique dirigée, le traitement d'échantillons, la chimie combinatoire, la séparation chirale, la protection de groupement, le déplacement d'équilibre, les médicaments polymériques et l'encapsulation.

Les nanosphères polymériques selon l'invention, notamment organisées à l'état de nanogel et/ou d'hydrogel, les nanogels selon l'invention et les hydrogels selon l'invention s'avèrent particulièrement utiles pour l'isolement sélectif dans leur coeur hydrophobe de molécules cibles sensibles en milieu aqueux ou dans des liquides biologiques complexes, comme par exemple certains métabolites ou des molécules dégradables par des enzymes.

Comme autre application des nanosphères polymériques selon l'invention, des nanogels selon l'invention et des hydrogels selon l'invention, on peut également citer la reconnaissance et l'extraction de molécules cibles hydrophobes ou amphiphiles en milieu organique. La plupart des macromonomères utilisables dans la présente invention sont en effet également solubles dans les solvants organiques, permettant ainsi le maintien en solution des nanosphères polymériques, et donc des matériaux à empreintes moléculaires selon l'invention.

### Définition

- Dans le cadre de la présente invention, on entend désigner par "molécule cible" toute entité capable de se lier spécifiquement à l'empreinte moléculaire, et par "molécule patron" toute molécule pouvant être utilisée à titre de patron pour la préparation de l'empreinte moléculaire.

La molécule patron peut être analogue à la molécule cible, et en particulier de taille moléculaire similaire. Les molécules cibles et les molécules patrons considérées dans la présente invention sont plus particulièrement hydrophobes ou amphiphiles.

Ainsi, selon un mode de réalisation de l'invention, l'empreinte formée dans le coeur des nanosphères polymériques selon invention est celle d'une molécule hydrophobe ou amphiphile.

Selon une variante, la molécule patron est identique à la molécule cible.

Selon une autre variante, la molécule patron est différente de la molécule cible mais est telle qu'elle forme une empreinte moléculaire possédant au moins un site de reconnaissance pour au moins une molécule cible.
- Par "site de reconnaissance ", on entend désigner le site de la matrice de l'empreinte moléculaire qui intervient effectivement dans la reconnaissance de la (ou des) molécule(s) cible(s),
- Par interaction entre la molécule patron ou la molécule cible et un site de reconnaissance s'entend, au sens de l'invention, la formation de liaisons faibles (par exemple de type liaisons de Van der Waals, liaisons hydrogène, liaisons pi donneur- pi accepteur, ou interactions hydrophobiques) et/ou de liaisons fortes (par exemple de type liaisons ioniques, liaisons covalentes, liaisons iono-covalentes, liaisons datives, ou encore de type liaisons de coordination).
- Par "nature hydrophile", on entend qualifier une aptitude à être solubilisé dans l'eau. Un composé ou segment hydrophile peut par exemple porter des fonctions capables de former des liaisons hydrogènes avec des molécules d'eau, comme par exemple les fonctions hydroxyle, amine, amide, ou acide carboxylique.
- Dans le cadre de la présente invention, une entité est qualifiée comme ayant une nature "hydrophile" lorsqu'elle présente un log P dans l'eau, à 25 °C, inférieur ou égal à 0.

Le log P est un paramètre communément utilisé pour estimer l'hydrophilie (respectivement l'hydrophobie) d'un composé chimique.

Il repose sur le coefficient de partage P, défini comme étant le rapport de la concentration dudit composé sous forme de molécule neutre dans une phase lipophile donnée, appelé solvant de partage, sur la concentration de ce même composé dans une phase aqueuse, dans un mélange biphasique constitué du solvant de partage et de la phase aqueuse, à une température et à un pH donnés.

Le solvant de partage classiquement utilisé pour évaluer le coefficient de partage est l'octanol. Il est toutefois possible de sélectionner un solvant organique présentant un comportement différent tel par exemple le cyclohexane, le chloroforme, ou encore le propylène glycol dipelargonate.

Dans la présente demande, les valeurs de log P indiquées sont calculées par modélisation ou mesurées en prenant pour solvant de partage l'octanol, à une température de 25 °C et un pH tel que le composé soit sous la forme d'une molécule neutre.

Les valeurs de log P peuvent être notamment calculées de façon théorique par des logiciels de chimie informatique, à l'image par exemple du logiciel Advanced Chemistry Development (ACD/Labs) V8.14.
- Dans le cadre de la présente invention, une entité est qualifiée comme ayant une "nature hydrophobe" lorsqu'elle présente un log P dans l'eau, à 25 °C, supérieur ou égal à 0.

A titre d'exemple de monomères hydrophobes au sens de l'invention, on peut notamment citer le 3-phénoxy-2-hydroxypropylacrylate (log P = 1,978±0,295), le 2-vinyl pyridine (log P = 1,338±0,264), le 2-carboxyéthyle méthacrylate (log P = 1,152±0,279), le styrène (log P = 2,700±0,191), l'acide méthacrylique (log P = 0,831±0,269) le méthacrylate de méthyle (log P = 1,346±0,250) ou encore le 2-(diméthylamino)éthyl acrylate (log P = 0,948±0,276), les log P indiqués étant ceux calculés par le logiciel Advanced Chemistry Development (ACD/Labs) V8.14.

A titre d'agents de réticulation hydrophobes au sens de l'invention, on peut notamment citer l'éthylène glycol diméthacrylate (log P = 2,783±0,342), le divinyl benzène (log P = 3,181±0,209), ou encore le triméthylolpropane triméthacrylate (log P = 3,154±0,453), les log P indiqués étant ceux calculés par le logiciel Advanced Chemistry Development (ACD/Labs) V8.14.
- Dans le cadre de la présente invention, une entité dite "amphiphile" présente au moins un motif hydrophobe et au moins un segment hydrophile.
- Au sens de la présente invention on entend désigner par "polymère" un produit issu de la polymérisation ou de la copolymérisation d'au moins des monomères et caractérisé par certaines propriétés telles que la masse moléculaire.
- Au sens de la présente invention, le terme "monomère" couvre une molécule capable d'être convertie en un polymère par combinaison avec elle-même ou avec d'autres molécules du même type, comme par exemple un macromonomère.
- Au sens de la présente invention on entend désigner par "macromonomère" une macromolécule polymérique apte à copolymériser constituée, à au moins une de ses extrémités, d'un motif polymérisable lui permettant de réagir comme un monomère, sur lequel est greffée de façon covalente une macromolécule pendante, linéaire ou ramifiée.

### NANOSPHERES POLYMERIOUES

Les nanosphères polymériques selon l'invention sont des particules sensiblement sphériques de nature polymérique, de taille pouvant variant de 1 nm à 10 micron environ, de préférence de 1 nm à 1 micron environ, voire de 5 à 500 nm. La taille moyenne en nombre des particules selon l'invention peut être évaluée par diffusion de la lumière.

Comme précisé précédemment, les nanosphères polymériques selon l'invention possèdent une structure en étoile de type "coeur-branches", cette architecture tridimensionnelle étant formée d'une entité de nature polymérique réticulée et hydrophobe constituant le "coeur", et de segments hydrophiles liés de façon covalente sur la surface extérieure du coeur, appelés "branches".

Les branches sont donc liées de façon covalente au coeur.

Les branches de nature hydrophile peuvent par exemple avoir un poids moléculaire variant de 300 à 300 000 g/mol et de préférence de 500 g/mol à 50 000 g/mol.

Les branches de nature hydrophile peuvent comprendre des segments hydrophiles choisis parmi les segments de type polyoxyéthylène (POE), polysaccharide, polyoxyéthylène-polyoxypropyléne-polyoxyéthylène (POE-PPO-POE), polyoxypropylène-polyoxyéthylène-polyoxypropylène (PPO-POE-PPO), polyvinylalcool, polydioxalane, poly(N-isopropylacrylamide) (poly(NIPAM)) polyéthylène imine, polyzwitterion, poly(méth)acrylamide, poly(amino alkyl(méth)acrylate), polyvinyl pyrrolidone, polypropylène glycol, polynucléotide, de polypeptide, polyélectrolyte tel que polysulfonique, polycarboxylique, polyphosphate et leurs copolymères hydrophiles.

Selon une variante de réalisation, les branches peuvent également comprendre au moins un segment présentant une LCST (Low Critical Solution Temperature), comme par exemple le poly(NIPAM), en une quantité suffisante pour pouvoir avantageusement conférer aux nanosphères qui les intègrent une aptitude à passer d'un état solide à un état liquide, lorsque la température atteint une certaine valeur critique. Cette propriété peut être particulièrement avantageuse pour isoler les nanosphères.

Les branches peuvent également comporter en outre au sein de leur(s) segment(s) polymérique(s) une ou plusieurs liaisons labiles susceptibles de se rompre sous l'action de la température, d'une variation de pH, d'une réaction d'oxydo-réduction, d'un faisceau d'ultrasons, ou d'un cisaillement.

Selon un premier mode de réalisation, les nanosphères selon l'invention peuvent être mises en oeuvre sous une forme individualisée c'est-à-dire sans qu'aucun lien ne soit établi entre elles. Une telle organisation implique notamment un défaut de réactivité entre les segments hydrophiles figurant les branches des structures en étoile. En particulier, les nanosphères conformes à l'invention peuvent se présenter sous la forme d'un nanogel, et peuvent par exemple être en dispersion dans l'eau.

Selon un second mode de réalisation, les nanosphères selon l'invention peuvent être organisées en un réseau de manière à former un hydrogel, dans lequel par exemple les cours polymériques hydrophobes qui forment les empreintes moléculaires figurent des noeuds de réticulation dudit hydrogel. Dans ce type d'organisation, des branches hydrophiles établissent des liens entre deux cours distincts.

Concernant cette seconde variante de réalisation, il peut être avantageux que les branches hydrophiles possèdent une liaison labile. Ainsi, la rupture de telles liaisons labiles au sein des branches hydrophiles peut entraîner l'individualisation des nanosphères, initialement organisées sous la forme d'un réseau et notamment d'un hydrogeL Les nanosphères selon l'invention peuvent alors se retrouver à nouveau sous une forme individualisée en dispersion dans la phase continue, et notamment à l'état d'un nanogel tel que décrit précédemment.

### PROCEDE DE PREPARATION DES NANOSPHERES

Comme précisé précédemment, la structure en étoile de type coeur-branches des nanosphères polymériques selon l'invention peut, selon un premier mode de réalisation de l'invention, être obtenue par copolymérisation d'au moins un macromonomère amphiphile avec au moins un monomère hydrophobe en présence d'au moins un agent de réticulation hydrophobe et d'au moins une molécule patron suivie de l'extraction de ladite molécule patron hors des nanosphères ainsi formées, par exemple par lavage.

La polymérisation peut être conduite par voie radicalaire, ionique, par polycondensation, par coordination, ou encore par voie sol-gel.

La polymérisation peut de préférence être réalisée selon une technique de polymérisation en phase dispersée, par exemple en phase continue aqueuse.

Cette technique de polymérisation permet d'obtenir des particules de taille allant du nanomètre au micron, la taille des particules dépendant notamment des quantités relatives de milieux dispersant et dispersé ainsi que du mode de dispersion.

Bien évidemment, la taille des nanosphères polymériques pourra être avantageusement adaptée par des méthodes connues de l'homme de métier en fonction de la taille de la molécule cible dont on souhaite former l'empreinte.

### Macromonomères amphiphiles

Les macromonomères amphiphiles utilisables selon la présente invention sont des macromolécules amphiphiles dont au moins une des extrémités porte un motif hydrophobe polymérisable, appelé encore motif hydrophobe apte à copolymériser dans la présente invention.

Les branches des nanosphères polymériques conformes à l'invention peuvent ainsi être formées des segments hydrophiles des macromonomères selon l'invention.

Les macromonomères amphiphiles considérés selon l'invention s'avèrent particulièrement avantageux comparativement aux agents tensioactifs non ioniques. Ainsi, à la différence des agents tensioactifs classiques, les segments hydrophiles des macromonoméres qui assurent la stabilisation des nanosphères polymériques, sont liés de façon covalente au coeur hydrophobe formant l'empreinte moléculaire.

Ces macromonomères permettent ainsi la formation de micelles en milieu aqueux, via leurs segments hydrophiles qui sont solubles dans l'eau tandis que leur motif polymérisable hydrophobe participe à la formation du coeur de la nanosphère à stabiliser.

Les macromonomères utilisables dans la présente invention possèdent de préférence une masse moléculaire moyenne en poids allant de 300 à 300 000 g/mol, en particulier allant de 500 à 50 000 g/mol.

Les segments hydrophiles des macromonomères utilisables dans la présente invention répondent à la définition proposée pour les branches hydrophiles décrites ci-dessus.

Ainsi, ils peuvent notamment être de type polyoxyéthylène (POE), polysaccharide, polyoxyéthylène-polyoxypropylène-polyoxyéthylène (POE-PPO-POE), polyoxypropylène-polyoxyéthylène-polyoxypropylène (PPO-POE-PPO), polyvinylalcool, polydioxalane, poly(N-isopropylacrylamide) (poly(NIPAM)), éthylène imine, polyzwitterion, poly(méth)acrylamide, poly(amino alkyl(méth)acrylate), polyvinyl pyrrolidone, polypropylène glycol, polynucléotide, polypeptide, polyélectrolyte tel que polysulfonique, polycarboxylique, polyphosphate et leurs copolymères hydrophiles ou tout autre polymère hydrosoluble modifié par une ou plusieurs fonctions hydrophobes polymérisables.

Comme motif hydrophobe apte à coploymériser pouvant être présent dans un macromonomère selon la présente invention, on peut notamment citer les motifs vinyliques, acryliques, méthacryliques, allyliques, styréniques ou tout autre motif insaturé susceptible de réagir par voie radicalaire, ainsi que les groupements chimiques permettant une réaction de polycondensation ou de sol gel.

Il est entendu que le macromonomère amphiphile peut comprendre, outre au moins un segment hydrophile et au moins un motif polymérisable hydrophobe, d'autres segments sous réserve que ceux-ci n'affectent pas sa nature amphiphile nécessaire à la formation de la structure étoile des nanosphères dérivant de sa copolymérisation. Ainsi, le macromonomère amphiphile convenant à la mise en oeuvre de ce mode de réalisation de l'invention peut également comprendre, en plus du motif polymérisable décrit ci-dessus, un segment hydrophobe adjacent audit motif polymérisable et du type polystyrène, polyalkyle, polyaryle, poly(méthylstyrène), polyuréthane, polychlorure de vinyle, polyimide, polyacétate de vinyle, polyester, polyoxypropylène, esters poly(méth)acrylique ou encore polyamide, étant entendu que les segments hydrophiles du macromonomère permettent de conférer aux branches correspondantes les propriétés requises selon l'invention

Selon une première variante, au moins un macromonomère amphiphile utilisable pour la mise en oeuvre de la présente invention, et en particulier utilisable pour la synthèse des nanosphères polymériques selon l'invention, peut posséder un unique motif hydrophobe apte à copolymériser, présent par exemple à l'une des extrémités du segment hydrophile. Les nanosphères polymériques alors obtenues, se présentent sous une forme individualisée, par exemple sous la forme d'un nanogel.

A titre d'exemple de macromonomères amphiphiles possédant un unique motif polymérisable utilisables dans la présente invention, on peut notamment citer le polyéthylène glycol éthyl éther méthacrylate, le polyéthylène glycol (méth)acrylate, le polyéthylène glycol méthyl éther-bloc-polylactide, le polyéthylène glycol alkyl éther (méth)acrylate, le polyéthylène glycol aryl éther (méth)acrylate, le polyéthylène glycol vinyl benzène, les copolymères à blocs comportant un segment hydrophile et un motif hydrophobe polymérisable tels que le polyacide acrylique-bloc-polystyryle styrène, le polyacrylamide-bloc-polystyryle styrène, le polysaccharide-bloc-polyméthacryloyle (méth)acrylate, ainsi que tout copolymère tribloc comportant un segment hydrophobe, un segment hydrophile et un unique motif hydrophobe polymérisable.

Les macro monomères amphiphiles possédant un unique motif polymérisable préférés dans cette variante de réalisation de l'invention sont le polyéthylène glycol (méth)acrylate et le polyéthylène glycol alkyl éther (méth)acrylate.

Selon une seconde variante, au moins un macromonomère amphiphile utilisable pour la mise en oeuvre de la présente invention, et en particulier utilisable pour la synthèse des nanosphères polymérique selon l'invention, peut posséder au moins deux motifs hydrophobes aptes à copolymériser, par exemple fixés à chacune des extrémités d'un segment hydrophile.

Les nanosphères polymériques sont alors obtenues sous une forme organisée en un réseau de manière à former un hydrogel dans lequel les coeurs hydrophobes dans lesquels sont formées les empreintes moléculaires figurent des noeuds de réticulation et les segments hydrophiles établissent des liens covalents entre deux coeurs distincts.

A titre d'exemple de macromonomères amphiphiles possédant au moins deux motifs polymérisables utilisables dans la présente invention, on peut notamment citer le polyéthylène glycol di(méth)acrylate, le polyéthylène glycol divinylbenzène, ou encore les polymères triblocs constitués d'un bloc central hydrophile (par exemple à base de polyéthylène glycol, d'acide polyacrylique, de polyacrylamide, de poly(vinylpyrrolidone), ou de polysaccharide et d'un bloc hydrophobe modifié par une fonction polymérisable à chaque extrémité (par exemple du type polystyryle styrène, ou polyméthacryloyle (méth)acrylate).

Les macromonomères amphiphiles possédant au moins deux motifs polymérisables préférés dans cette variante de réalisation de l'invention sont le polyéthylène glycol diacrylate et le polyéthylène glycol diméthacrylate.

Il est entendu que la synthèse d'un macromonomère convenant à la réalisation de l'invention relève des compétences de l'homme de métier.

Selon cette variante, au moins un macromonomère amphiphile possédant un unique motif hydrophobe apte à copolymériser, par exemple tels que décrits précédemment peut être en outre copolymérisé lors de la synthèse des nanosphères polymériques.

En d'autres termes, les nanosphères polymériques selon l'invention peuvent résulter notamment de la copolymérisation d'au moins un macromonomère amphiphile possédant un unique motif polymérisable et d'au moins un macromonomère amphiphile possédant au moins deux motifs polymérisable.

C'est le cas en particulier des nanosphères polymériques se présentant sous une forme organisée en un réseau de manière à former un hydrogel.

Les segments hydrophiles des macromonomères amphiphiles possédant un unique motif polymérisable peuvent alors avantageusement être utiles pour stabiliser les nanosphères.

Cette stabilisation permet ainsi avantageusement de s'affranchir de la nécessité d'utiliser d'autres systèmes de stabilisation, comme par exemple des agents tensioactifs.

D'autre part, l'ajout de ces macromonomères amphiphiles possédant un unique motif polymérisable permet également d'agir sur les propriétés physiques du gel tel que l'élasticité.

Ainsi, la teneur en macromonomères amphiphiles possédant un unique motif polymérisable par rapport aux macromonomères amphiphiles possédant au moins deux polymérisables est optimisée en fonction des propriétés physiques de l'hydrogel et des propriétés des nanosphères désirées.

Ces macromonomères amphiphiles possédant un unique motif polymérisable peuvent notamment être présents dans une teneur comprise entre 0 et 50 % en poids par rapport au poids total en macromonomères amphiphiles possédant au moins deux motifs polymérisables

Comme précisé précédemment, le macromonomère amphiphile considéré est copolymérisé avec au moins un monomère hydrophobe en présence d'au moins un agent de réticulation hydrophobe, et d'au moins une molécule patron.

Les monomères hydrophobes convenant à la mise en oeuvre de la présente invention possèdent également avantageusement des affinités avec la molécule patron dont on veut faire l'empreinte.

Ils peuvent par exemple être choisis parmi les monomères acryliques, méthacryliques, acrylamides, styréniques, vinyliques, allyliques ou tout autre monomère insaturé, ainsi que les groupements chimiques permettant une réaction de polycondensation ou de sol-gel.

Les monomères hydrophobes convenant à la mise en oeuvre de la présente invention présentent un log P supérieur à 0, et de préférence supérieur à 0,5, voire supérieur à 1.

A titre d'exemple de monomères hydrophobes utilisables dans le cadre de la présente invention, on peut notamment citer le méthacrylate de méthyle, le styrène, l'éthylstyrène, l'acide méthacrylique, les méthacrylates d'alkyle, les acrylates d'alkyle, les acrylates d'allyle, les méthacrylates d'allyle, les acrylates d'aryle, les méthacrylates d'aryle, les dérivés du styrène, l'acétate de vinyle, l'acrylonitrile, le méthacrylonitrile, le méthacrylate de 2-amino éthyle, le méthacrylate de t-amylle, le méthacrylate de 2-(1-aziridinyl)éthyle, le t-butylacrylamide, l'acrylate de butyle, le méthacrylate de butyle, la 4-vinyl pyridine, la 2-vinyl pyridine, la 2-vinyl quinoléine, le diméthylamino éthyl acrylate, le 3-phénoxy-2-hydroxypropylacrylate et le 2-carboxyéthyle méthacrylate.

Selon un mode de réalisation particulier, les nanosphères résultent de la copolymérisation d'au moins un macromonomère méthacrylate de polyéthylèneglycol et d'au moins un monomère de type (méth)acrylate.

Selon un mode de réalisation particulier, un monomère hydrophobe apte à copolymériser avec le macromonomère amphiphile peut en outre être lié de façon covalente à la molécule cible, ou à la molécule patron par une liaison labile du type ester, disulfure, amide, ester boronique, cétal, hémicétal, carbamate, silyle, éther, thioester ou thioéther. Après synthèse de la nanosphère et formation de l'empreinte moléculaire, cette liaison peut être rompue par une variation de pH, une réaction d'oxydoréduction, ou par une réaction avec un acide ou une base de Lewis, conduisant alors à la libération et à l'élimination de la molécule patron.

Selon un autre mode de réalisation, les nanosphères résultent de la copolymérisation d'au moins un macromonomère, d'au moins un monomère et d'au moins un agent de réticulation de log P supérieur à 0 en présence d'une molécule patron de log P supérieur à 0, en phase grasse dispersée dans une solution aqueuse.

Selon un autre mode de réalisation, les nanosphères résultent de la copolymérisation d'au moins un macromonomère, et d'au moins un monomère de log P supérieur à 0,5 et d'au moins un agent de réticulation de log P supérieur à 0,75 en présence d'une molécule patron de log P supérieur à 0, en phase grasse dispersée dans une solution aqueuse.

Selon un autre mode de réalisation, les nanosphères résultent de la copolymérisation d'au moins un macromonomère, d'au moins un monomère et d'au moins un agent de réticulation de log P supérieur à 1 en présence d'une molécule patron de log P supérieur à 0 en phase grasse dispersée dans une solution aqueuse.

La structure en étoile de type coeur-branche caractérisant les nanosphères polymériques selon l'invention peut également être obtenue par d'autres voies de synthèse que celle exposée ci-dessus.

Par exemple, cette synthèse peut être conduite en deux étapes successives, la première étape étant destinée à la formation d'une entité polymérique hydrophobe et réticulée formant l'empreinte moléculaire d'une molécule patron, et la seconde étape à la transformation en surface de cette entité avec des entités de nature hydrophile.

Ainsi, selon un autre de ses aspects, l'invention concerne un procédé de préparation de nanosphères polymériques selon l'invention comprenant :
- une première étape de copolymérisation d'au moins un monomère hydrophobe fonctionnalisé en présence d'au moins un agent de réticulation hydrophobe, d'au moins une molécule patron, et éventuellement d'au moins un monomère hydrophobe tel que décrit précédemment,
- une deuxième étape de transformation en surface de l'entité polymérique hydrophobe et réticulée obtenue à l'issue de la première étape avec une ou plusieurs entités hydrophiles,
- et l'extraction de ladite molécule patron hors des nanosphères ainsi formées, par exemple par lavage.

Dans la mesure où la surface externe de l'entité réticulée obtenue à l'issue de la première étape doit être transformée afin de comprendre des entités hydrophiles nécessaires pour conférer à la structure finale un comportement amphiphile, la première étape doit mettre en oeuvre au moins un monomère hydrophobe fonctionnalisé.

Par "fonctionnalisé", on entend désigner, au sens de la présente invention, un monomère comportant, en outre de son (ses) motif(s) polymérisable(s) mis en jeu au cours de la synthèse de l'entité polymérique hydrophobe réticulée, au moins une autre fonction non impliquée dans la formation de ladite entité hydrophobe, et susceptible de réagir ultérieurement avec un composé différent du monomère auquel elle est liée.

Le terme "fonctionnalisé" peut également être utilisé pour désigner l'entité polymérique hydrophobe résultant de la copolymérisation de tels monomères, et présentant également sur sa surface des fonctions réactives.

Ces fonctions peuvent être du type de toutes les fonctions réactives classiquement rencontrées en chimie organique, comme par exemple la fonction alcool, acide, amine, halogénure d'acide, halogénure d'alkyle, vinylique, anhydride, amide ou encore uréthane.

A titre d'exemple de monomères hydrophobes fonctionnalisés, on peut ainsi notamment citer le 1,6-heptadièn-4-ol, le 1-hexèn-3-ol, le 1,5-hexadièn-3,4-diol, le chlorométhyl styrène, le bromométhyl styrène, le méth(acrylate) d'aminoéthyle, le méth(acrylate) d'hydroxyéthyle et le divinyl benzène.

Selon un mode de réalisation de l'invention l'entité polymériques hydrophobe est issue de la polymérisation d'au moins un monomère hydrophobe de log P supérieur à 0 et d'au moins un agent de réticulation de log P supérieur à 0, en présence d'une molécule patron de log P supérieur à 0.

En ce qui concerne la deuxième étape visant à conférer un comportement amphiphile à l'entité hydrophobe, réticulée obtenue à l'issue de la première étape, elle peut être effectuée selon plusieurs alternatives.

Selon une première alternative, l'entité polymérique hydrophobe portant au moins une fonction réactive est mise en présence d'au moins une macromolécule de nature hydrophile fonctionnalisée, notamment formée d'au moins une chaîne principale linéaire, le cas échéant ramifiée, porteuse à au moins l'une de ses extrémités d'une fonction apte à réagir avec au moins une fonction de la surface de l'entité polymérique hydrophobe réticulée.

Selon cette alternative, la transformation selon la deuxième étape comprend au moins un greffage covalent, notamment par condensation, d'au moins une macromolécule de nature hydrophile fonctionnalisée en surface de l'entité hydrophobe et réticulée issue de la première étape. Ces macromolécules de nature hydrophile peuvent être formées de segment(s) hydrophile(s) conforme(s) à la définition proposée ci-dessus pour les macromonomères amphiphiles, sous réserve qu'ils soient aptes en tant que tels à réagir avec la fonction réactive de la surface de l'entité hydrophobe ou aient été au préalable fonctionnalisés à cette fin.

Ainsi, les macromolécules de nature hydrophile peuvent être formées de segments hydrophiles de type polyoxyéthylène (POE), polysaccharide, polyoxyéthylène-polyoxypropylène-polyoxyéthylène (POE-PPO-POE), polyoxypropylène-polyoxyéthylène-polyoxypropylène (PPO-POE-PPO), polyvinylalcool, polydioxalane, poly(N-isopropylacrylamide) (poly(NIPAM)), éthylène imine, polyzwitterion, poly(méth)acrylamide, poly(amino alkyl(méth)acrylate), polyvinyl pyrrolidone, polypropylène glycol, polynucléotide, polypeptide, polyélectrolyte tel que polysulfonique, polycarboxylique, polyphosphate et leurs copolymères hydrophiles, et comportent en outre, à au moins l'une de leurs extrémités, une fonction susceptible de réagir avec l'entité hydrophobe fonctionnalisée, par exemple par condensation.

Les fonctions susceptibles de réagir avec l'entité polymérique fonctionnalisée sont par exemple toutes les fonctions réactives classiquement rencontrées en chimie organique, comme par exemple la fonction alcool, acide aminé, halogénure d'acide, anhydride, amide ou encore uréthane.

A titre d'exemple de macromolécule de nature hydrophile fonctionnalisée, on peut notamment citer le polyéthylène glycol amine et le polyéthylène glycol mercaptan.

Selon une seconde alternative, l'entité hydrophobe obtenue à l'issue de la première étape est mise en présence d'au moins un monomère hydrophile fonctionnalisé. Selon cette seconde variante, la deuxième étape comprend en outre au moins une réaction de polymérisation d'au moins un monomère hydrophile fonctionnalisé en surface de l'entité polymérique hydrophobe et réticulée obtenue à l'issue de la première étape de manière à former des segments hydrophiles aptes à conférer à la structure étoile finale un comportement amphiphile.

Les monomères hydrophiles convenant à la transformation en surface du coeur hydrophobe doivent avoir un log P inférieur à 0.

A titre d'exemples de monomères hydrophiles fonctionnalisés convenant à la mise en oeuvre de cette seconde variante de réalisation, on peut notamment citer l'oxyde d'éthylène, les éthers cycliques, les lactones, les lactames, les amines cycliques, les acrylates cationiques, anioniques ou zwitterioniques, le styrène sulfonate, des esters poly(méth)acryliques, le méthacrylamide, l'acrylamide, l'acide 2- acrylamidoglycolique, l'acide 2-acrylamido-2-méthyl propane sulfonique, le N-acryloxysuccinimide, le N-acryloyl pyrrolidinone, le N-(3-aminopropyl)méthacrylamide, le N,N'-diméthylacrylamide, le 2-méthylène-1,3- propanediol, le vinyl méthyl sulfone, l'acide vinyl phosphonique ou encore le sel de sodium de l'acide vinyl sulfonique.

Bien entendu, l'homme de l'art est à même de sélectionner les composés convenables pour synthétiser des nanosphères selon l'invention suivant les différentes variantes de réalisation décrites ci-dessus.

Par ailleurs, toute combinaison des modes de réalisation décrits ci-dessus est considérée comme variante de mise en oeuvre de la présente invention.

Quelque soit le mode de préparation considéré, à savoir *via* une unique étape impliquant la copolymérisation d'un monomère hydrophobe avec un macromonomère amphiphile ou via deux étapes selon le procédé précisé ci-dessus, la formation du coeur hydrophobe requiert la présence simultanée d'un agent de réticulation hydrophobe et d'au moins une molécule patron.

En effet, le coeur polymérique hydrophobe est avantageusement réticulé, ce qui lui permet notamment de conserver la forme de l'empreinte quelles que soient les conditions opératoires. L'agent de réticulation doit être de nature hydrophobe, de sorte à être incorporé dans le coeur des nanosphères au cours du processus de polymérisation.

L'agent de réticulation convenant à la mise en oeuvre de la présente invention présente un log P supérieur à 0, de préférence supérieur à 0,75, voire supérieur à 1.

A titre d'agents de réticulation utilisables dans le cadre de la présente invention, on peut notamment citer le divinylbenzène (DVB), le 1,3 diisopropènyl benzène (DIP), le diméthacrylate d'éthylène glycol (EGDMA), le diméthacrylate de tétraméthylène (TDMA), le *N,O*-bisacryloyl-L-phénylalaninol, le 1,4-phénylène diacrylamide, le *N,N'-*1,3-phénylénebis(2-méthyl-2-propenamide) (PDBMP), le 1,4;3,6-dianhydro-D-sorbitol-2,5-diméthacrylate, le isopropoylénébis(1,4-phénylène) diméthacrylate, le triméthylolpropane triméthacrylate (TRIM), le pentaérythritol triacrylate (PETRA), le pentaérythritol tétraacrylate (PETEA), les acrylates difonctionnels, les méthacrylates difonctionnels, les acrylates trifonctionnels, les méthacrylates trifonctionnels, les acrylates tétrafonctionnels, les méthacrylates tétrafonctionnels, les alkylène glycol diacrylates, les alkylène glycol méthacrylates, le dicarbonate de diallyldiglycol, le diallyl maléate, le diallyl fumarate, le diallyl itaconate, le divinyl oxalate, le divinyl malonate, le diallyl succinate, le bis-phénol A diméthacrylate, le bis-phénol A diacrylate, les di (alkène) amines, le triméthylol propane triacrylate, le divinyl éther, la divinyl sulfone, et le diallyl phthalate.

L'agent de réticulation est présent dans le milieu réactionnel en une proportion allant de 0,05 % à 30 % en poids, de préférence allant de 0,10% à 10% en poids.

### Molécules patrons

Comme précisé précédemment, cette polymérisation ou copolymérisation peut être conduite par amorçage radicalaire, par polycondensation ou par sol-gel en présence d'au moins un agent de réticulation et d'au moins une molécule patron.

Selon une première variante de l'invention, la molécule patron peut être initialement présente, lors de la synthèse des nanosphères polymériques selon l'invention, sous forme libre en solution.

Selon une seconde variante de l'invention, la molécule patron peut être initialement liée de façon covalente à au moins un monomère utilisé pour la synthèse des nanosphères polymériques selon l'invention, et être libérée ensuite une fois lesdites nanosphères polymériques formées, par rupture de ladite liaison covalente.

La liaison covalente entre ledit monomère et ladite molécule patron peut notamment être une liaison labile susceptible de se rompre sous l'action de la température, d'une variation de pH, d'une réaction d'oxydo-réduction, d'un faisceau d'ultrasons, ou d'un cisaillement, à l'image par exemple d'une liaison ester, disulfure, ou encore amide.

En d'autres termes, il est possible, selon cette seconde variante, d'utiliser en outre pour la synthèse des nanosphères polymériques selon l'invention, des monomères spécifiques selon la (ou les) molécule(s) cible(s) visée(s), et en particulier des monomères dérivés d'une molécule patron jouant ainsi en partie le rôle de monomères destinés à former le coeur de nature polymérique, et en partie le rôle de la molécule patron. Autrement dit, une partie de ces monomères, une fois la polymérisation terminée, a vocation à être éliminée de sorte à donner naissance aux sites de reconnaissance.

Par suite, la reconnaissance moléculaire peut être obtenue par des interactions covalentes et/ou non covalentes pour une même molécule cible. Ainsi, pour une molécule cible unique, l'interaction avec l'empreinte polymérique peut se dérouler au moins en deux sites distincts du site de reconnaissance comme cela est par exemple divulgué dans Wulff G. et al., Macromol. Chem. Phy. 1989, 190, 1717 et 1727.

Selon un mode de réalisation, des procédés de préparation des nanosphères polymériques selon l'invention, d'un nanogel selon l'invention, et d'un hydrogel selon l'invention, la copolymérisation peut mettre en oeuvre au moins un monomère lié à la molécules patron par au moins une liaison covalente, de préférence labile.

Lorsque la molécule dont on souhaite faire l'empreinte est hydrophobe, elle est entièrement soluble dans la phase grasse dispersée de l'émulsion, et la réaction de polymérisation décrite ci-dessus se déroulé tout autour de sa surface, conduisant à l'empreinte de la molécule dans son ensemble.

En revanche, si la molécule dont on souhaite faire l'empreinte est amphiphile, c'èst-à-dire comporte une partie hydrophile et une partie hydrophobe, à l'image d'un tensioactif par exemple, alors seule la partie hydrophobe de la molécule sera présente dans la phase dispersée, et par conséquent, la réaction de polymérisation décrite ci-dessus ne se déroulera qu'autour de la surface de cette partie hydrophobe, conduisant à l'empreinte de seulement une partie de la molécule cible, ou de la molécule patron.

Les molécules patrons, correspondant ou non à la molécule cible, covenant à la mise en oeuvre de la présente invention présentent avantageusement un log P supérieur à 0. Elles peuvent être hydrophobes ou amphiphiles.

La molécule dont on souhaite faire l'empreinte peut par ailleurs être de toute taille et de toute masse moléculaire. Il peut ainsi notamment s'agir d'une molécule de masse moléculaire comprise entre 50 et 2000 g/mol, ou encore d'une macromolécule de masse moléculaire pouvant par exemple aller jusqu'à 100000 g/mol.

Elle peuvent notamment être choisies parmi des polyaromatiques hétérocycliques, des pesticides, des molécules ayant des propriétés organoleptiques tels que les parfums et les arômes, des colorants alimentaires tels que les caroténoïdes, les riboflavines, la tartrazine ou l'amarante, des molécules biologiquement actives à caractère thérapeutique ou pharmaceutique, des molécules antioxydantes telles que les polyphénols ou la flavonoïde, des colorants tels que les colorants azoïques, l'anthraquinonique, la polyméthinique, la phtalocyanine.

Selon un mode de réalisation de l'invention, la molécule cible, ou la molécule patron est une molécule biologiquement active, et notamment à caractère thérapeutique ou pharmaceutique.

Comme molécule biologiquement active, on peut par exemple citer les molécules hypnotiques, anxiolitiques, anti cancéreuses, myorelaxants, diurétiques, anti-ulcéreuses, anti-épiléptiques, antibiotiques, anesthésiques, analgésiques, antihistaminiques, antihypertensifs, antihyperthyroidique, anti inflammatoire, antimigraineux, antimuscarinique, antiostéoporotiques, antiparkinsonien, antiprotozoaire, antipsychotique, antiseptiques, antispasmodique, antithrombotique, antituberculeux, bronchodilatateur, cardiotonique, cholinergique, stimulant du système nerveux central, les neurotransmetteurs, contraceptives, immunomodulateur, immunosuppresseur, laxatifs, agent bloquant, antiarrhythmiques, les tocophérols, stéroïdes ou stérols.

La molécule cible considérée selon la présente invention peut notamment être choisie parmi des polypeptides, des hormones, des cytokines, des enzymes, des protéines de toutes masses moléculaires telles que les anticorps, l'albumine, la fibrinogène, la fibronectine, l'insuline ou la fétoprotéine.

Selon un mode de réalisation de l'invention, la molécule cible, ou la molécule patron, est choisie parmi des polypeptides, des hormones, des enzymes, des cytokines et des protéines, et notamment des protéines de masse moléculaire inférieure à 65 000 g/mol, et notamment inférieure à 50 000 g/mol, voire inférieure à 5 000 g/mol.

Selon un autre mode de réalisation de l'invention, la molécule cible, ou la molécule patron, présente une masse moléculaire inférieure ou égale à 2000 g/mol, notamment inférieure ou égale à 1500 g/mol, voire même inférieure ou égale à 1000 g/moL

La réalisation des opérations de polymérisation ou de polycondensation considérées selon l'invention relève clairement des compétences de l'homme de l'art. En particulier, le choix du solvant, de la température, du pH et les conditions d'amorçage constituent des opérations de routine pour l'homme de métier.

Par exemple, le solvant pour la polymérisation peut être hydrophobe et porogène, comme par exemple le toluène, le benzène, le dichlorométhane ou le chloroforme, peut éventuellement être utilisé pour former la phase dispersée.

En ce qui concerne l'amorçage, il peut être réalisé suivant tout procédé connu de l'homme de l'art, et dépendra de la technique de polymérisation sélectionnée (radicalaire, ionique, par polycondensation, par coordination ou par sol-gel).

### APPLICATIONS

Les nanosphères polymériques, les nanogels et les hydrogels selon la présente invention trouvent leur application d'une façon générale dans l'extraction, la séparation, la purification, la détection, l'absorption, l'adsorption, la rétention et la libération contrôlée.

Les nanosphères, les nanogels et les hydrogels selon l'invention trouvent ainsi notamment leur application dans le domaine de l'analyse dans le domaine de l'agroalimentaire, de la pharmacie, du biomédical, de l'alimentaire, de la défense ou de l'environnement.

Ils peuvent également trouver une application dans le domaine des capteurs, tels que biocapteurs, dans le criblage moléculaire, la synthèse chimique dirigée, le traitement d'échantillon, la chimie combinatoire, la séparation chirale, la protection de groupement, la catalyse, le déplacement d'équilibre, les médicaments, polymériques, et l'encapsulation.

Ils peuvent également trouver une application dans le domaine des sciences de la vie, par la formation de particules furtives capable de résister à l'adsorption non spécifique des protéines et/ou de libérer des molécules biologiquement actives ou de cibler une partie spécifique des cellules ou d'un organe.

Comme cela a déjà été mentionné précédemment, la présente invention a également pour objet l'utilisation des nanosphères polymériques, des nanogels et des hydrogels selon l'invention à des fins d'isolement sélectif de molécules cibles sensibles en milieux aqueux, ou dans des liquides biologiques complexes

La présente invention concerne aussi l'utilisation des nanosphères polymériques, des nanogels et des hydrogels selon l'invention à des fins de reconnaissance de molécules cibles hydrophobes ou amphiphiles en milieu organique.

Les exemples ci-après sont donnés à titre illustratif et sans caractère limitatif de l'invention.

Dans les exemples ci-après, les molécules patron sont identiques aux molécules cibles visées.

Les log de P indiqués dans les exemples ci-après ont été calculés par le logiciel Advanced Chemistry Development (ACD/Labs) V8.14.

### Exemple 1 : Synthèse de nanosphères selon l'invention sous la forme d'un nanogel

Deux types de nanogels test sont préparés, l'un en utilisant à titre de molécule patron le Boc Tyrosine (log P = 2,64), et l'autre du propranolol (log P = 3,10), selon le protocole opératoire suivant.

Dans un tube à essai, 3,00 g de PEG méthacrylate (M = 2000 g/mol, 50 % dans l'eau) sont dilués dans 3,2 g d'eau pour conduire à une solution transparente. 30 mg de triméthacrylate triméthylol proprane (log P = 3,15) et 3,2 mg de 3-phénoxy-2-hydroxypropylacrylate (log P = 1,98) sont alors ajoutés sous forte agitation pour conduire à une dispersion dudit agent de réticulation.

Le pH est ensuite ajusté de sorte que la molécule patron soit la plus hydrophobe dans l'eau. Ainsi, le pH est ajusté à 11 dans le cas du propranolol qui est insoluble dans l'eau en milieu basique, alors qu'il vaut 4 lorsqu'on utilise la Boc Tyrosine (log P= 2,64).

9 mg de la molécule patron considérée sont alors ajoutés dans l'échantillon test, tandis qu'un échantillon témoin constitué du même milieu réactionnel à l'exception d'une molécule cible est réalisé.

Après bullage d'azote pendant 10 minutes, 2,2 mg de persulfate de sodium sont ajoutés, puis le milieu réactionnel est mis sous agitation pendant quelques minutes jusqu'à obtention d'une mise en dispersion des agents de réticulation et des monomères. Puis le milieu réactionnel est chauffé à 60°C une nuit pour conduire à la formation d'un gel homogène et trouble.

Les gels obtenus sont ensuite lyophilisés et la taille des nanogels est mesurée avec un nanosizer.

Le nanogel témoin présente une taille moyenne de 200 nm tandis que les deux nanogels test possèdent une taille moyenne de 80 mm rendant compte de l'interaction de la molécule cible considérée avec le coeur du nanogel.

La molécule cible est extraite par lavage dans une solution aqueuse suivie d'une précipitation dans l'éthylène glycol à froid.

### Exemple 2 : Synthèse de nanosphères selon l'invention organisées en réseau de manière à former un hydrogel

Deux types de nanogels test sont préparés en utilisant les mêmes molécules cibles que pour l'Exemple 1, selon le protocole opératoire suivant.

Dans un flacon de 2 mL, 0,15 g de polyéthylène glycol PEG soit 0,105 g de diacrylate 700 et 0,045 g de monométhacrylate de polyéthylèneglycol de masse molaire 2000 g/mol, sont introduits pour conduire, après mélange, à une solution micellaire homogène transparente dans laquelle les micelles mesurent environ 2 nm. 36 mg de triméthacrylate de triméthylol proprane sont alors introduits conduisant à la formation d'un système biphasique. 15 mg de 2-(diméthylamino) éthylacrylate (log P = 0,95) et 23 mg de 3-phénoxy-2-hydroxypropylacrylate (log P = 1,98) sont alors ajoutés sous forte agitation pour conduire à une dispersion des monomères.

Le pH est ajusté tel que décrit en exemple 1, selon la nature de la molécule cible utilisée.

30 mg de molécule cible sont alors ajoutés dans l'échantillon test, tandis qu'un échantillon témoin constitué du même milieu réactionnel à l'exception de la molécule cible est réalisé.

Après bullage d'azote pendant 10 minutes, 2,29 mg de persulfate de sodium sont ajoutés, puis le milieu réactionnel est mis sous agitation pendant quelques minutes jusqu'à obtention d'une mise en dispersion des agents de réticulation et des monomères. Puis le milieu réactionnel est chauffé à 60 °C une nuit pour conduire à la formation d'un gel homogène et trouble.

Les gels sont ensuite lavés plusieurs fois à l'eau puis deux fois en utilisant une solution de 5 % dans l'acide acétique et sont enfin rincés encore plusieurs fois avec de l'eau, permettant l'extraction de plus de 95 % de la molécule cible.

### Exemple 3 : Caractérisation du taux d'adsorption de nanosphères selon l'invention.

Des gels test et témoin de Boc Tyrosine sont obtenus selon un protocole similaire à celui mis en oeuvre dans l'exemple 1, en utilisant comme monomères un mélange de 3-phénoxy-2-hydroxypropylacrylate et de carboxyéthylacrylate (log P = 0,6). Ces gels subissent ensuite les mêmes étapes d'extraction de la molécule cible que celle décrites dans l'exemple 2, de sorte à extraire plus de 95 % de Boc Tyrosine.

Les deux gels (test et témoin) sont séchés et placés dans une solution aqueuse de Boc Tyrosine (126 µg/ml). Les pourcentages de Boc tyrosine adsorbés en fonction du temps sont présentés dans le Tableau 1 ci-dessous.

**Tableau 1 : pourcentage d'adsorption de la Boc tyrosine en fonction du temps pour une concentration initiale (126 µg/mL).**

| | Témoin | MIP |
|---|---|---|
| Temps (h) | % adsorbée | % adsorbée |
| 0 | 0 | 0 |
| 0,75 | 10,0 | 33,3 |
| 1,5 | 13,3 | 43,2 |
| 2,5 | 15,3 | 45,4 |
| 5 | 22,0 | 53,7 |
| 8,5 | 23,9 | 54,2 |
| 23 | 25,3 | 59,1 |

Le gel test adsorbe la molécule cible beaucoup plus fortement et plus rapidement que le gel témoin. L'empreinte moléculaire réalisée selon l'invention présente ainsi une affinité importante pour la molécule cible tandis que l'adsorption non spécifique observée sur le témoin est faible.

### Exemple 4 : Caractérisation de la capacité de relargage des nanosphères selon l'invention

Les échantillons utilisés en Exemple 3 sont séchés puis placés dans deux solutions aqueuses de 2,5 mL, respectivement. Des prélèvements de ces solutions sont effectués à intervalles réguliers pour déterminer le pourcentage de relargage de Boc Tyrosine rapporté à la quantité de Boc Tyrosine adsorbée au cours de l'évaluation du taux d'adsorption décrite en Exemple 3. Les pourcentages de relargage pour les gels test et témoin figurent dans le Tableau 2 ci-dessous.

**Tableau 2 : pourcentage de Boc tyrosine relarguée en fonction du temps**

| | MIP | témoin |
|---|---|---|
| temps (h) | % relargué | % relargué |
| 0 | 0 | 0. |
| 0,7 | 32,5 | 66,1 |
| 1,5 | 38,7 | 74,5 |
| 3 | 48,0 | 79,1 |
| 5,5 | 53,4 | 91,9 |
| 8 | 53,6 | 95,2 |

Le pourcentage de Boc Tyrosine relarguée est toujours plus faible pour le gel test que pour le gel témoin, la différence pouvant atteindre jusqu'à 40 %.

Ainsi la cinétique de relargage est plus lente pour le gel test que pour le gel témoin.

### Exemple 5 : Synthèse de nanosphères polymériques conformes à l'invention suivant le procédé de préparation en deux étapes

### 1- Première étape de formation de l'entité polymérique hydrophobe et réticulée

La synthèse est effectuée dans un réacteur muni d'un dispositif d'agitation, d'une circulation d'argon, d'un thermomètre et de tubes gradués contenant les différents réactifs purifiés.

150ml de THF sont tout d'abord introduits dans le réacteur. Le milieu réactionnel est ensuite refroidi à -10 °C, puis 3,2 g de butyl lithium, 10 g de divinylbenzène (log P = 3,18), 2 g d'éthylstyrène (log P = 3,70) et 0,75 g d'anthracène (molécule cible) sont introduits à leur tour dans le réacteur, et la polymérisation se déroule pendant 30 minutes, à -10 °C.

### 2- Deuxième étape de transformation en surface de ladite entité polymérique hydrophobe et réticulée par une entité hydrophile

Sous un vide statique à -30 °C, 100 g d'oxyde éthylène sont ajoutés au milieu réactionnel. Le milieu réactionnel est alors réchauffé jusqu'à atteindre la température ambiante, et la polymérisation se déroule pendant 48h à 30 °C. L'oxyde d'éthylène polymérise, selon un processus anionique, sur les sites réactifs portés en surface de l'entité polymérique. A la fin de la réaction, quelques gouttes d'un mélange de méthanol et d'acide sont ajoutées, à 30 °C, pour désactiver les espèces vivantes. La solution obtenue est ensuite filtrée sur papier, centrifugée, à nouveau filtrée sur papier puis précipitée dans de l'heptane ou de l'éther.

### Exemple 6 : Synthèse d'un hydrogel dégradable dont les branches formant la structure étoile sont des segments triblocs de POE présentant une séquence centrale de poly(1,3-dioxolane) (PDXL)

### 1. Synthèse du PDXL.

On prépare du PDXL de masse moléculaire en poids 2000 mol/g par réaction de 2,87 moles de 1,3-DXL avec 0,12 mole de Et(OH)₂ et 2,9 x 10⁻⁴ mole de CF₃SO₃H, sous atmosphère d'azote selon le protocole suivant. Le 1,3-DXL est tout d'abord introduit, puis la solution d'acide triflique dans le chlorure de méthylène est ajoutée. La température augmente jusqu'à 21 °C. Le mélange réactionnel est ensuite désactivé par addition d'une solution comprenant 500 mg de sodium dans 10 ml du méthanol.

Après filtration et évaporation du solvant, le produit obtenu est dissout dans du dichlorométhane.

Le polymère ainsi obtenu est précipité dans 1,5 L de méthanol à - 40 °C. Sa masse moléculaire moyenne en nombre est déterminée par CES et par RMN¹⁹F.

### 2. Synthèse d'un copolymère et macromonomère de POE à séquence centrale de PDXL.

10g du PDXL obtenu à l'issue de la première étape sont lyophilisés dans du benzène puis dissout, sous atmosphère contrôlée, dans 50 mL de THF. L'addition de 10 mL de diphénylméthyl potassium, à température ambiante, permet alors la transformation des extrémités hydroxyle du PDXL en groupements alcoolates. Le milieu réactionnel est ensuite maintenu sous agitation pendant environ deux heures puis 7,5g d'oxyde d'éthylène sont ajoutés, et la polymérisation de l'oxyde d'éthylène amorcée par les alcoolates formés lors de la précédente étape débute. Le milieu réactionnel revient ensuite à température ambiante, puis il est chauffé à 30 °C pendant environ 48h.

On additionne alors 1 g de chlorure de méthacryloyle pour fonctionnaliser le copolymère ainsi obtenu.

La masse moléculaire moyenne en nombre du copolymère et du macromonomère fonctionnel sont 3500, caractérisés par CES dans le THF. Le taux de doubles liaisons de macromonomère peut être déterminé quant à lui par spectroscopie UV.

Les copolymères ainsi formés peuvent être utilisés pour la synthèse d'hydrogels au titre de macromonomères difonctionnels dégradables. Par suite, la dégradation par hydrolyse des fragments PDXL desdits hydrogels peut conduire à la formation de nanogels.

### Exemple 7 : Synthèse de nanosphères à plus grande échelle selon l'invention sous la forme d'un nanogel et leurs purifications

Des nanogels tests sont préparés en utilisant à titre de molécule patron le Boc phénylalanine anilide (log P = 4,4) selon le protocole opératoire suivant.

367 mg de molécule patron sont mélangés avec 355 mg d'acide méthacrylique (log P = 0,83) et 2,66 g d'éthylène glycol diméthacrylate (log P= 2,78). A cette solution est additionnée 22 g de PEG méthacrylate (M = 2000 g/mol, 50 % dans l'eau) pour former une solution trouble qui est agitée avec 100 g d'eau. La solution, dont le pH est de l'ordre de 4, est introduite dans un réacteur à double enveloppe de 200 mL régulée à 60°C. L'agitation est produite par une turbine à une vitesse de 1000 tpm.

Une solution témoin est réalisée de la même façon en absence de molécules patrons.

Après bullage d'azote pendant 20 minutes, l'agitation est poursuivie pendant trente minutes jusqu'à obtention d'une solution uniformément trouble. L'addition de 360 mg de V50 entame la réaction de polymérisation qui dure toute la nuit. Une solution uniformément trouble et stable, plus visqueuse que la solution initiale est obtenue.

La solution de nanogel est purifiée par dialyse en milieu aqueux avec de la cellulose régénérée possédant un seuil de coupure de 6000-8000.

La taille des nanogels obtenus est mesurée avec un nanosizer.

Le nanogel témoin est constitué de particules dont 65 % ont une taille moyenne de 600 nm et 35 % ont une taille moyenne de 35 nm. Le nanogel test est constitué de particules dont 30% ont une taille moyenne de 300 nm et 70% ont une taille moyenne de 30 nm, rendant compte de l'interaction de la molécule cible considérée avec le coeur du nanogel.

La solution de nanogel est ensuite lyophilisée pour donner une poudre blanche.

Pour l'extraction de la molécule patron, la poudre est dispersée dans un volume d'acétonitrile puis précipitée dans dix volumes d'éther refroidi à -40°C. Le polymère précipité est isolé par filtration. Cette opération est répétée jusqu'à ce qu'il n'y ait plus de molécules patrons extractibles.

Une analyse du taux d'adsorption obtenue par séparation des molécules cibles liées des molécules cibles libres par filtration à travers une membrane (seuil de coupure 5000) au cours d'une centrifugation, confirme la forte affinité de l'empreinte préparée pour la molécule cible.

## Revendications

1. Nanosphères polymériques réticulées possédant une structure en étoile de type coeur-branches, dans laquelle les branches sont de nature hydrophile et le coeur est de nature polymérique, réticulé, hydrophobe et forme l'empreinte de tout ou partie d'au moins une molécule cible.

2. Nanosphères selon la revendication 1, dans lesquelles l'empreinte est celle d'une molécule hydrophobe ou amphiphile.

3. Nanosphères selon la revendication 1 ou 2, dans lesquelles les branches sont liées de façon covalente au coeur.

4. Nanosphères selon l'une quelconque des revendications 1 à 3, dans lesquelles les branches de nature hydrophile comprennent des segments hydrophiles choisis parmi les segments de type polyoxyéthylène (POE), polysaccharide, polyoxyéthylène-polyoxypropylène-polyoxyéthylène (POE-PPO-POE), polyoxypropylène-polyoxyéthylène-polyoxypropylène (PPO-POE-PPO), polyvinylalcool, polydioxalane, poly(N-isopropylacrylamide) (poly(NIPAM)) polyéthylène imine, polyzwitterion, poly(méth)acrylamide, poly(amino alkyl(méth)acrylate), polyvinyl pyrrolidone, polypropylène glycol, polynucléotide, de polypeptide, polyélectrolyte tel que polysulfonique, polycarboxylique, polyphosphate et leurs copolymères hydrophiles.

5. Nanosphères selon l'une quelconque des revendications 1 à 4, dans lesquelles les branches sont formées par le segment hydrophile d'au moins un macromonomère amphiphile et le coeur dérive de la copolymérisation du motif polymérisable hydrophobe dudit macromonomère amphiphile avec au moins un monomère hydrophobe, en présence d'au moins un agent de réticulation hydrophobe et d'au moins une molécule patron.

6. Nanosphères selon la revendication 5, dans lesquelles le motif hydrophobe apte à copolymériser est choisi parmi les motifs vinyliques, acryliques, allyliques, styréniques ou tout autre motif insaturé susceptible de réagir par voie radicalaire, ainsi que les groupements chimiques permettant une réaction de polycondensation ou de sol gel.

7. Nanosphères selon la revendication 5 ou 6, dans lesquelles au moins un macromonomère amphiphile possède un unique motif hydrophobe apte à copolymériser et. est notamment choisi parmi le polyéthylène glycol éthyl éther méthacrylate, le polyéthylène glycol (méth)acrylate, le polyéthylène glycol méthyl éther-bloc-polylactide, le polyéthylène glycol alkyl éther (méth)acrylate, le polyéthylène glycol aryl éther (méth)acrylate, le polyéthylène glycol vinyl benzène, les copolymères blocs comportant un segment hydrophile et un motif hydrophobe polymérisable tels que le polyacide acrylique-bloc-polystyryle styrène, le polyacrylamide-bloc-polystyryle styréne et le polysaccharide-bloc-polyméthacryloyle (méth)acrylate.

8. Nanosphères selon la revendication 5 ou 6, dans lesquelles au moins un macromonomère amphiphile possède au moins deux motifs hydrophobes aptes à copolymériser, et est notamment choisi parmi le polyéthylène glycol di(meth)acrylate, le polyéthylène glycol divinylbenzène, ou encore les polymères triblocs constitués d'un bloc central hydrophile (par exemple à base de polyéthylène glycol, d'acide polyacrylique, de polyacrylamide, de poly(vinylpyrrolidone), ou de polysaccharide et d'un bloc hydrophobe modifié par une fonction polymérisable à chaque extrémité (par exemple du type polystyryle styrène, ou polyméthacryloyle(méth)acrylate).

9. Nanosphères selon l'une quelconque des revendications 5 à 8, dans lesquelles le monomère hydrophobe est choisi parmi les monomères acryliques, méthacryliques, acrylamides, styréniques, vinyliques, allyliques ainsi que les groupements chimiques permettant une réaction de polycondensation ou de sol-gel.

10. Nanosphères selon l'une quelconque des revendication 5 à 9 **caractérisées en ce que** le macromonomère amphiphile est un méthacrylate de polyéthylène glycol et le monomère hydrophobe est de type (méth)acrylate.

11. Nanosphères selon l'une quelconque des revendications précédentes, dans lesquelles la molécule cible, ou la molécule patron, présente une masse moléculaire inférieure ou égale à 2000 g/mol, et notamment inférieure ou égale à 1500 g/mol, voire même inférieure ou égale à 1000 g/mol.

12. Nanogel **caractérisé en ce qu'**il se présente sous la forme d'une dispersion de nanosphères polymériques selon l'une quelconque des revendications 1 à 11.

13. Hydrogel **caractérisé en ce qu'**il se présente sous la forme d'un réseau de nanosphères polymériques selon l'une quelconque des revendications 1 à 11 dont les coeurs polymériques hydrophobes figurent des noeuds de réticulation dudit hydrogel.

14. Procédé de préparation de nanosphères polymériques selon l'une quelconque des revendication 1 à 11, par copolymérisation d'au moins un macromonomère amphiphile avec au moins un monomère hydrophobe en présence d'au moins un agent de réticulation hydrophobe et d'au moins une molécule patron, suivie de l'extraction de ladite molécule patron hors desdites nanosphères polymériques.

15. Procédé de préparation d'un nanogel selon la revendication 12, comprenant au moins la copolymérisation d'au moins un macromonomère amphiphile comprenant un unique motif hydrophobe apte à copolymériser avec au moins un monomère hydrophobe en présence d'au moins un agent de réticulation hydrophobe et d'au moins une molécule patron, et l'extraction de ladite molécule patron hors desdites nanosphères polymériques.

16. Procédé de préparation d'un hydrogel selon la revendication 13, comprenant au moins la copolymérisation d'au moins un macromonomère amphiphile comprenant au moins deux motifs hydrophobes aptes à copolymériser avec au moins un monomère hydrophobe en présence d'au moins un agent de réticulation hydrophobe et d'au moins une molécule patron, et l'extraction de ladite molécule patron hors desdites nanosphères polymériques.

17. Procédé selon l'une quelconque des revendications 14 à 16, dans lequel le monomère hydrophobe est tel que défini dans la revendication 9.

18. Procédé de préparation de nanosphères polymériques selon l'une quelconque des revendications 1 à 4 comprenant :
- une première étape de copolymérisation d'au moins un monomère hydrophobe fonctionnalisé en présence d'au moins un agent de réticulation hydrophobe, d'au moins une molécule patron, et éventuellement d'au moins un monomère hydrophobe, notamment tel que défini dans la revendication 9.
- une deuxième étape de transformation en surface de l'entité polymérique hydrophobe et réticulée obtenue à l'issue de la première étape avec une ou plusieurs entités hydrophiles, et
- l'extraction de ladite molécule patron hors desdites nanosphères polymériques.

19. Procédé selon la revendication 18, **caractérisé en ce que** la deuxième étape comprend au moins un greffage covalent d'au moins une macromolécule de nature hydrophile fonctionnalisée, en surface de ladite entité polymérique hydrophobe et réticulée.

20. Procédé selon la revendication 18, **caractérisé en ce que** la deuxième étape comprend au moins une réaction de polymérisation d'au moins un monomère hydrophile fonctionnalisé en surface de ladite entité polymérique hydrophobe.

21. Procédé selon la revendication 20, **caractérisé en ce que** le monomère hydrophile fonctionnalisé est choisi parmi l'oxyde d'éthylène, les éthers cycliques, les lactones, les lactames, les amines cycliques, les acrylates cationiques, anioniques ou zwitterioniques, le styrène sulfonate, des esters poly(méth)acryliques, le méthacrylamide, l'acrylamide, l'acide 2- acrylamidoglycolique, l'acide 2-acrylamido-2-méthyl propane sulfonique, le N-acryloxysuccinimide, le N-acryloyl pyrrolidinone, le N-(3-aminopropyl)méthacrylamide, le N,N'-diméthylacrylamide, le 2-méthylène-1,3-propanediol, le vinyl méthyl sulfone, l'acide vinyl phosphonique ou encore le sel de sodium de l'acide vinyl sulfonique.

22. Procédé selon l'une quelconque des revendications 14 à 21, dans lequel la copolymérisation met en oeuvre au moins un monomère lié à la molécule patron par au moins une liaison covalente, de préférence labile.

23. Utilisation de nanosphères polymériques selon l'une quelconque des revendications 1 à 11, d'un nanogel selon la revendication 12, ou d'un hydrogel selon la revendication 13 à des fins d'extraction, de détection, de séparation, de purification, d'absorption, d'adsorption, de rétention ou de libération contrôlée ou encore dans des applications choisies parmi les capteurs, la catalyse de réactions chimiques, le criblage de molécules, la synthèse chimique dirigée, le traitement d'échantillons, la chimie combinatoire, la séparation chirale, la protection de groupement, le déplacement d'équilibre, les médicaments polymériques et l'encapsulation.

24. Utilisation de nanosphères polymériques selon l'une quelconque des revendications 1 à 11 d'un nanogel selon la revendication 12 ou d'un hydrogel selon la revendication 13 à des fins d'isolement sélectif de molécules cibles sensibles en milieux aqueux ou dans des liquides biologiques complexes.

25. Utilisation de nanosphères polymériques selon l'une quelconque des revendications 1-à 11 d'un nanogel selon la revendication 12 ou d'un hydrogel selon la revendication 13 à des fins de reconnaissance et d'extraction de molécules cibles hydrophobes ou amphiphiles en milieu organique.

## Claims

1. Crosslinked polymeric nanospheres having a star-shaped structure of the core-branch type, in which the branches are of a hydrophilic nature and the core is of a polymeric, crosslinked, hydrophobic nature and forms the imprint of all or at least part of a target molecule.

2. Nanospheres according to claim 1, in which the imprint is that of a hydrophobic or amphiphilic molecule.

3. Nanospheres according to claim 1 or 2, in which the branches are covalently bonded to the core.

4. Nanospheres according to any one of claims 1 to 3, in which the branches of a hydrophilic nature comprise hydrophilic segments chosen from segments of the polyoxyethylene (POE), polysaccharide, polyoxyethylene-polyoxypropylene-polyoxyethylene (POE-PPO-POE), polyoxypropylene-polyoxyethylene-polyoxypropylene (PPO-POE-PPO), polyvinyl alcohol, polydioxalane, poly(N-isopropylacrylamide) (poly(NIPAM)), polyethyleneimine, polyzwitterion, poly(meth)acrylamide, poly(aminoalkyl (meth)acrylate), polyvinylpyrrolidone, polypropylene glycol, polynucleotide, polypeptide and polyelectrolyte, such as polysulphonic, polycarboxylic and polyphosphate, type and their hydrophilic copolymers.

5. Nanospheres according to any one of claims 1 to 4, in which the branches are formed by the hydrophilic segment of at least one amphiphilic macromonomer and the core is derived from the copolymerization of the hydrophobic polymerizable motif of the said amphiphilic macromonomer with at least one hydrophobic monomer, in the presence of at least one hydrophobic crosslinking agent and at least one master molecule.

6. Nanospheres according to claim 5, in which the hydrophobic motif capable of copolymerizing is chosen from vinyl, acrylic, methacrylic, allyl and styrene motifs or any other unsaturated motif capable of reacting by the free-radical route, and the chemical groups allowing a polycondensation or sol-gel reaction.

7. Nanospheres according to claim 5 or 6, in which at least one amphiphilic macromonomer has a single hydrophobic motif capable of copolymerizing and is chosen in particular from polyethylene glycol ethyl ether methacrylate, polyethylene glycol (meth)acrylate, polyethylene glycol methyl ether-block-polylactide, polyethylene glycol alkyl ether (meth)acrylate, polyethylene glycol aryl ether (meth)acrylate, polyethylene glycol vinylbenzene and block copolymers containing a hydrophilic segment and a polymerizable hydrophobic motif, such as polyacrylic acid-block-polystyrylstyrene, polyacrylamide-block-polystyrylstyrene and polysaccharide-block-polymethacryloyl (meth)acrylate.

8. Nanospheres according to claim 5 or 6, in which at least one amphiphilic macromonomer has at least two hydrophobic motifs capable of copolymerizing and is chosen in particular from polyethylene glycol di(meth)acrylate, polyethylene glycol divinylbenzene, or triblock polymers consisting of a hydrophilic central block (for example based on polyethylene glycol, polyacrylic acid, polyacrylamide, poly(vinylpyrrolidone), or polysaccharide) and of a hydrophobic block modified by a polymerizable function at each end (for example of the polystyryl styrene or polymethacryloyl (meth)acrylate type).

9. Nanospheres according to any one of claims 5 to 8, in which the hydrophobic monomer is chosen from acrylic, methacrylic, acrylamide, styrene, vinyl and allyl monomers, and chemical groups allowing a polycondensation or sol-gel reaction.

10. Nanospheres according to any one of claims 5 to 9, **characterized in that** the amphiphilic macromonomer is a polyethylene glycol methacrylate and the hydrophobic monomer is of the (meth)acrylate type.

11. Nanospheres according to any one of the preceding claims, in which the target molecule, or the master molecule, has a molecular weight of less than or equal to 2,000 g/mol, and in particular less than or equal to 1,500 g/mol, or even less than or equal to 1,000 g/mol.

12. Nanogel, **characterized in that** it is in the form of a dispersion of polymeric nanospheres according to any one of claims 1 to 11.

13. Hydrogel, **characterized in that** it is in the form of a network of polymeric nanospheres according to any one of claims 1 to 11, of which the hydrophobic polymeric cores represent crosslinking nodes of the said hydrogel.

14. Process for the preparation of polymeric nanospheres according to any one of claims 1 to 11 by copolymerization of at least one amphiphilic macromonomer with at least one hydrophobic monomer in the presence of at least one hydrophobic crosslinking agent and at least one master molecule, followed by extraction of the said master molecule from the said polymeric nanospheres.

15. Process for the preparation of a nanogel according to claim 12, comprising at least the copolymerization of at least one amphiphilic macromonomer comprising a single hydrophobic motif capable of copolymerizing with at least one hydrophobic monomer in the presence of at least one hydrophobic crosslinking agent and at least one master molecule, and extraction of the said master molecule from the said polymeric nanospheres.

16. Process for the preparation of a hydrogel according to claim 13, comprising at least the copolymerization of at least one amphiphilic macromonomer comprising at least two hydrophobic motifs capable of copolymerizing with at least one hydrophobic monomer in the presence of at least one hydrophobic crosslinking agent and at least one master molecule, and extraction of the said master molecule from the said polymeric nanospheres.

17. Process according to any one of claims 14 to 16, in which the hydrophobic monomer is as defined in claim 9.

18. Process for the preparation of polymeric nanospheres according to any one of claims 1 to 4, comprising:
- a first step of copolymerization of at least one functionalized hydrophobic monomer in the presence of at least one hydrophobic crosslinking agent, at least one master molecule, and optionally at least one hydrophobic monomer, in particular as defined in claim 9,
- a second step of conversion on the surface of the hydrophobic and crosslinked polymeric entity obtained at the end of the first step with one or more hydrophilic entities, and
- extraction of the said master molecule from the said polymeric nanospheres.

19. Process according to claim 18, **characterized in that** the second step comprises at least a covalent grafting of at least one macromolecule of a functionalized hydrophilic nature on the surface of the said hydrophobic and crosslinked polymeric entity.

20. Process according to claim 18, **characterized in that** the second step comprises at least a polymerization reaction of at least one functionalized hydrophilic monomer on the surface of the said hydrophobic polymeric entity.

21. Process according to claim 20, **characterized in that** the functionalized hydrophilic monomer is chosen from ethylene oxide, cyclic ethers, lactones, lactams, cyclic amines, cationic, anionic or zwitterionic acrylates, styrene sulphonate, poly(meth)acrylic esters, methacrylamide, acrylamide, 2-acrylamidoglycollic acid, 2-acrylamido-2-methylpropanesulphonic acid, N-acryloxysuccinimide, N-acryloylpyrrolidinone, N-(3-aminopropyl)methacrylamide, N,N'-dimethylacrylamide, 2-methylene-1,3 propanediol, vinyl methyl sulphone, vinylphosphonic acid or the sodium salt of vinylsulphonic acid.

22. Process according to any one of claims 14 to 21, in which the copolymerization uses at least one monomer bonded to the master molecule by at least one covalent, preferably labile bond.

23. Use of polymeric nanospheres according to any one of claims 1 to 11, a nanogel according to claim 12 or a hydrogel according to claim 13 for the purpose of extraction, detection, separation, purification, absorption, adsorption, retention or controlled release or in applications chosen from sensors, catalysis of chemical reactions, screening of molecules, directed chemical synthesis, treatment of samples, combinatory chemistry, chiral separation, group protection, displacement of equilibrium, polymeric medicaments and encapsulation.

24. Use of polymeric nanospheres according to any one of claims 1 to 11, a nanogel according to claim 12 or a hydrogel according to claim 13 for the purpose of selective isolation of sensitive target molecules in aqueous media or in complex biological liquids.

25. Use of polymeric nanospheres according to any one of claims 1 to 11, a nanogel according to claim 12 or a hydrogel according to claim 13 for the purpose of recognition and extraction of hydrophobic or amphiphilic target molecules in an organic medium.

## Patentansprüche

1. Vernetze, polymerische Nanokugeln, die eine sternförmige Struktur des Kern-Äste-Typs aufweisen, wobei die Äste von hydrophiler Natur sind und der Kern von polymerischer, vernetzter, hydrophober Natur ist und die Prägung des Ganzen oder eines Teils von mindestens einem Zielmolekül ausbildet.

2. Nanokugeln gemäß Anspruch 1, wobei die Prägung die eines hydrophoben oder amphiphilen Moleküls ist.

3. Nanokugeln gemäß Anspruch 1 oder 2, wobei die Äste kovalent an den Kern gebunden sind.

4. Nanokugeln gemäß einem beliebigen der Ansprüche 1 bis 3, wobei die Äste von hydrophiler Natur hydrophile Segmente umfassen, ausgewählt aus den Segmenten des Typs Polyoxyethylen (POE), Polysaccharid, Polyoxyethylen-Polyoxypropylen-Polyoxyethylen (POE-PPO-POE), Polyoxypropylen-Polyoxyethylen-Polyoxypropylen (PPO-POE-PPO), Polyvinylalkohol, Polydioxalan, Poly(N-isopropylacrylamid) (Poly(NIPAM)), Polyethylenimin, Polyzwitterion, Poly(meth)acrylamid, Poly(aminoalkyl(meth)acrylat), Polyvinylpyrrolidon, Polypropylenglykol, Polynukleotid, Polypeptid, Polyelektrolyt wie z.B. polysulfonisch, polycarboxylisch, Polyphosphat und ihren hydrophilen Copolymeren.

5. Nanokugeln gemäß einem beliebigen der Ansprüche 1 bis 4, wobei die Äste durch das hydrophile Segment mindestens eines amphiphilen Makromonomers gebildet werden und sich der Kern von der Copolymerisation des polymerisierbaren hydrophoben Motivs des amphiphilen Makromonomers mit mindestens einem hydrophoben Monomer in Gegenwart mindestens eines hydrophoben Vernetzungsmittels und mindestens eines Modellmoleküls (molecule patron) ableitet.

6. Nanokugeln gemäß Anspruch 5, wobei das zum Copolymerisieren fähige hydrophobe Motiv ausgewählt wird aus den vinylischen, acrylischen, methacrylischen, allylischen, styrenischen Motiven oder jedem anderen ungesättigtem Motiv, das im Stande ist, radikalisch zu reagieren, sowie den chemischen Gruppen, die eine Polykondensations- oder Sol-Gel-Reaktion erlauben.

7. Nanokugeln gemäß Anspruch 5 oder 6, wobei mindestens ein amphiphiles Makromonomer ein einzigartiges hydrophobes Motiv, das zum Copolymerisieren fähig ist, aufweist und insbesondere aus Polyethylenglykolethylethermethacrylat, Polyethylenglykol(meth)acrylat, Polyethylenglykolmethylether-Block-Polylactid, Polyethylenglykolalkylether(meth)acrylat, Poly-ethylenglykolarylether(meth)acrylat, Polyethylenglykolvinylbenzol, BlockCopolymeren, die ein hydrophiles Segment und ein hydrophobes polymersierbares Motiv beinhalten, wie z.B. Polyacrylsäure-Block-Polystyrylstyrol, Polyacrylamid-Block-Polystyrylstyrol und Polysaccharid-Block-Polymethacryloyl(meth)acrylat, ausgewählt wird.

8. Nanokugeln gemäß Anspruch 5 oder 6, wobei mindestens ein amphiphiles Makromonomer mindestens zwei hydrophobe Motive, die zum Copolymerisieren fähig sind, aufweist und insbesondere aus Polyethylenglykoldi(meth)acrylat, Polyethylenglykoldivinylbenzol, oder außerdem den Triblockpolymeren ausgewählt wird, die aus einem zentralen hydrophilen Block (z.B. basierend auf Polyethylenglykol, Polyacrylsäure, Polyacrylamid, Poly(vinylpyrrolidon) oder Polysaccharid) und einem hydrophoben Block bestehen, der durch eine polymerisierbare Funktion an jedem Endstück modifiziert ist (z.B. vom Typ Polystyrylstyrol oder Polymethacryloyl(meth)acrylat).

9. Nanokugeln gemäß einem beliebigen der Ansprüche 5 bis 8, wobei das hydrophobe Monomer aus Acryl-, Methacryl-, Acrylamid-, Styrol-, Vinyl-, Allylmonomeren sowie den chemischen Gruppen, die eine Polykondensations- oder Sol-Gel-Reaktion erlauben, ausgewählt wird.

10. Nanokugeln gemäß einem beliebigen der Ansprüche 5 bis 9, **dadurch gekennzeichnet, dass** das amphiphile Makromonomer ein Methacrylat von Polyethylenglykol ist und das hydrophobe Monomer vom Typ (Meth)acrylat ist.

11. Nanokugeln gemäß einem beliebigen der vorstehenden Ansprüche, wobei das Zielmolekül oder das Modellrnolekül eine molare Masse von weniger oder gleich 2000 g/mol und insbesondere weniger oder gleich 1500 g/mol, ja sogar weniger oder gleich 1000 g/mol aufweist.

12. Nanogel, **dadurch gekennzeichnet, dass** es in Form einer Dispersion von polymerischen Nanokugeln gemäß einem beliebigen der Ansprüche 1 bis 11 auftritt.

13. Hydrogel, **dadurch gekennzeichnet, dass** es in Form eines Netzes von polymerischen Nanokugeln gemäß einem beliebigen der Ansprüche 1 bis 11 auftritt, deren hydrophobe polymerischen Kerne Vernetzungsknoten des Hydrogels darstellen.

14. Verfahren zum Herstellen von polymerischen Nanokugeln gemäß einem beliebigen der Ansprüche 1 bis 11 durch Copolymerisation mindestens eines amphiphilen Makromonomers mit mindestens einem hydrophoben Monomer in Gegenwart mindestens eines hydrophoben Vernetzungsmittels und mindestens eines Modellmoleküls, gefolgt von der Extraktion des Modellmoleküls aus den polymerischen Nanokugeln.

15. Verfahren zum Herstellen eines Nanogels gemäß Anspruch 12, umfassend mindestens die Copolymerisation mindestens eines amphiphilen Makromonomers, umfassend ein einzigartiges hydrophobes Motiv, das zum Copolymerisieren fähig ist, mit mindestens einem hydrophoben Monomer in Gegenwart mindestens eines hydrophoben Vernetzungsmittels und mindestens eines Modellmoleküls und die Extraktion des Modellmoleküls aus den polymerischen Nanokugeln.

16. Verfahren zum Herstellen eines Hydrogels gemäß Anspruch 13, umfassend mindestens die Copolymerisation mindestens eines amphiphilen Makromonomers, umfassend mindestens zwei hydrophobe Motive, die zum Copolymerisieren fähig sind, mit mindestens einem hydrophoben Monomer in Gegenwart mindestens eines hydrophoben Vernetzungsmittels und mindestens eines Modellmoleküls und die Extraktion des Modellmoleküls aus den polymerischen Nanokugeln.

17. Verfahren gemäß einem beliebigen der Ansprüche 14 bis 16, wobei das hydrophobe Monomer wie in z.B. Anspruch 9 definiert ist.

18. Verfahren zur Herstellung von polymerischen Nanokugeln gemäß einem beliebigen der Ansprüche 1 bis 4, umfassend:
- einen ersten Schritt der Copolymerisation mindestens eines funktionalisierten hydrophoben Monomers in Gegenwart mindestens eines hydrophoben Vernetzungsmittels, mindestens eines Modellmoleküls und gegebenenfalls mindestens eines hydrophoben Monomers, insbesondere wie in z.B. Anspruch 9 definiert,
- einen zweiten Schritt der Oberflächentransformation der hydrophoben und vernetzten polymerischen Einheit, die nach dem ersten Schritt erhalten wurde, mit einer oder mehreren hydrophilen Einheiten und
- die Extraktion des Modellmoleküls aus den polymerischen Nanokugeln.

19. Verfahren gemäß Anspruch 18, **dadurch gekennzeichnet, dass** der zweite Schritt mindestens ein kovalentes Pfropfen mindestens eines funktionalisierten hydrophilartigen Makromoleküls auf die Oberfläche der hydrophoben und vernetzten polymerischen Einheit umfasst.

20. Verfahren gemäß Anspruch 18, **dadurch gekennzeichnet, dass** der zweite Schritt mindestens eine Polymerisationsreaktion mindestens eines funktionalisierten hydrophilen Monomers auf der Oberfläche der hydrophoben polymerischen Einheit umfasst.

21. Verfahren gemäß Anspruch 20, **dadurch gekennzeichnet, dass** das funktionalisierte hydrophile Monomer aus Ethylenoxid, cyclischen Ethern, Lactonen, Lactamen, cyclischen Aminen, kationischen, anionischen oder zwitterionischen Acrylaten, Styrolsulfonat, Poly(meth)acrylestem, Methacrylamid, Acrylamid, 2-Acrylamidoglykolsäure, 2-Acrylamido-2-methylpropansulfonsäure, N-Acryloxysuccinimid, N-Acryloylpyrrolidinon, N-(3-Aminopropyl)methacrylamid, N,N'-Dimethylacrylamid, 2-Methylen-1,3-propandiol, Vinylmethylsulfon, Vinylphosphonsäure oder außerdem dem Natriumsalz von Vinylsulfonsäure ausgewählt wird.

22. Verfahren gemäß einem beliebigen der Ansprüche 14 bis 21, wobei die Copolymerisation mit mindestens einem Monomer durchgeführt wird, das an das Modellmolekül mittels mindestens einer kovalenten, insbesondere labilen, Bindung gebunden ist.

23. Verwendung von polymerischen Nanokugeln gemäß einem beliebigen der Ansprüche 1 bis 11, des Nanogels gemäß Anspruch 12 oder des Hydrogels gemäß Anspruch 13 zur Extraktion, Detektion, Auftrennung, Aufreinigung, Absorption, Adsorption, Retention oder kontrollierten Freisetzung oder außerdem in Applikationen, ausgewählt aus Sensoren, Katalyse von chemischen Reaktionen, Molekülsieben, direkter chemischer Synthese, Probenbehandlung, kombinatorischer Chemie, chiraler Auftrennung, Schutz von Gruppen, Gleichgewichtsverschiebung, polymerischen Medikamenten und Einkapselung.

24. Verwendung von polymerischen Nanokugeln gemäß einem beliebigen der Ansprüche 1 bis 11, des Nanogels gemäß Anspruch 12 oder des Hydrogels gemäß Anspruch 13 zum selektiven Isolieren von in wässrigen Milieus oder in komplexen biologischen Flüssigkeiten sensiblen Zielmolekülen.

25. Verwendung von polymerischen Nanokugeln gemäß einem beliebigen der Ansprüche 1 bis 11, des Nanogels gemäß Anspruch 12 oder des Hydrogels gemäß Anspruch 13 zur Wiedererkennung und Extraktion von hydrophoben oder amphiphilen Zielmolekülen in einem organischen Milieu.
